# FASCICULE DE BREVET EUROPEEN

(11) **EP 3 419 675 B1**
(45) Date de publication et mention de la délivrance du brevet: **15.12.2021**
(21) Numéro de dépôt: 17709147.7
(22) Date de dépôt: 13.02.2017
(51) Int. Cl.: A61L 9/12, B60H 3/00

(54) **PROCÉDÉ DE DIFFUSION DE FRAGRANCE DANS L'HABITACLE D'UN VEHICULE AUTOMOBILE A PERFORMANCE AMELIOREE**
BEDUFTUNGSVERFAHREN EINES KRAFTFAHRZEUGS MIT VERBESSERTER LEISTUNG
METHOD OF FRAGRANCING THE PASSENGER COMPARTMENT OF A MOTOR VEHICLE WITH IMPROVED PERFORMANCE

(30) Priorité: 24.02.2016 FR 1651484
(43) Date de publication de la demande: 02.01.2019
(73) Titulaire: PSA Automobiles SA, 78300 Poissy (FR)
(72) Inventeur: POCHOLLE, Benoit, 25260 ETOUVANS (FR); GHIBAUDO, Laurence, 90000 BELFORT (FR); ROIZOT,Didier, 25600 SOCHAUX (FR)
(86) Numéro de dépôt international: PCT/FR2017/050316
(87) Numéro de publication internationale: WO 2017/144796

(56) Documents cités:
- EP-A1- 2 145 788
- EP-A2- 2 095 980
- DE-C1- 19 955 035
- US-A1- 2012 107 172

## Description

### Domaine de l'invention

La présente invention est relative aux procédés de diffusion de fragrance à l'intérieur de l'habitacle des véhicules automobiles.

### Arrière-plan de l'invention

L'habitacle d'un véhicule automobile constitue un lieu clos de faible volume ne favorisant pas l'élimination des odeurs désagréables pouvant s'y dégager et causées notamment par les effluves de gaz d'échappement pénétrant par le système d'aération, les fumées de tabac, les restes de nourriture ou bien encore la présence d'animaux.

Pour éviter la persistance de ces odeurs, il est bien connu d'adapter sur les aérateurs des véhicules des dispositifs diffuseurs de fragrances comportant un récipient contenant un concentré de parfum liquide, et surmonté d'un bouchon diffuseur réglable de sorte à assurer une diffusion plus ou moins importante de ce parfum.

Le paramétrage de ces dispositifs est ainsi particulièrement sommaire et ces derniers présentent également l'inconvénient de dénaturer l'harmonie d'ensemble de la planche de bord et de dégrader le niveau de qualité perçu du véhicule.

Afin de palier à ces inconvénients, il est également connu d'équiper les véhicules automobiles de diffuseurs de fragrance intégrés.

La demande de brevet française FR 2 815 294 divulgue ainsi un tel dispositif diffuseur de fragrance monté en dérivation sur une canalisation de sortie d'air pulsé de l'installation de chauffage et de climatisation de l'habitacle d'un véhicule automobile. Le document EP2095980 décrit un dispositif d'amenée d'une matière odorante dans un habitacle d'un véhicule automobile, comprenant un support de matière odorante avec au moins une matière odorante, un canal d'air d'amenée d'air à enrichir au support de matière odorante et/ou de retour d'air enrichi du support de matière odorante, dans l'habitacle du véhicule automobile, l'au moins une matière odorante étant susceptible d'être amenée au canal d'air, une soufflante pour faire circuler de l'air dans le canal d'air et un dispositif de fermeture pour commander et/ou régler la quantité de l'au moins une matière odorante susceptible d'être amenée du support de matière odorante en fonction du temps, la quantité de matière odorante fournie en fonction du temps doit être commandée en fonction de la température effective de l'air circulant sur le support de matière odorante. Ce but est atteint par le fait qu'au moyen d'au moins un capteur de température, la température de l'air, à enrichir et/ou enrichi par au moins une matière odorante, se trouvant dans le canal d'air, est mesurable et, en fonction de la température mesurée, le dispositif d'obturation peut être commandé et/ou réglé.

Les brevets DE 199 55 035 C1, EP 2 145 788 A1 et US 2012/0107172 A1 sont aussi connus dans le domaine de la diffusion de fragrances dans un véhicule automobile.

Toutefois, avec ce type de dispositif, les fragrances odorantes ne peuvent être diffusées que lorsque le mode chauffage ou climatisation est activé, ce qui peut s'avérer particulièrement contraignant pour l'usager.

Afin d'améliorer leur souplesse d'utilisation, il est donc également connu de prévoir des dispositifs autonomes aptes à diffuser du parfum dans l'habitacle du véhicule indépendamment du système de ventilation et/ou de climatisation et au travers d'au moins un passage en forme de fente ménagé dans la paroi frontale de la planche de bord.

La demande de brevet FR 3 002 188 divulgue ainsi un tel dispositif diffuseur de fragrances comportant un boîtier situé dans la planche de bord du véhicule et doté d'un réceptacle apte à recevoir de manière amovible une cartouche interchangeable comprenant au moins un compartiment contenant un élément de stockage et de diffusion d'une fragrance (en l'espèce, une mèche imprégnée).

Le dispositif diffuseur comprend également un pulseur rotatif destiné à produire un flux d'air pulsé s'écoulant dans ce boîtier avant d'être diffusé dans une conduite communiquant avec l'intérieur de ce dernier et débouchant au niveau du passage ménagé dans la planche de bord de sorte à se répandre dans l'habitacle.

Le compartiment comporte un orifice dont l'ouverture est commandée par des moyens d'obturation de type à clapets, et à travers duquel peut s'effectuer l'échange à l'intérieur du boîtier entre le flux d'air généré par le pulseur et la fragrance.

Le pilotage d'un tel diffuseur autonome est assuré manuellement par le conducteur grâce à des organes de commandes permettant de l'activer, de choisir la fragrance délivrée (lorsque la cartouche comprend plusieurs compartiments contenant chacun des fragrances différentes) et éventuellement de régler le débit de diffusion.

Ce type de réglage succinct n'est cependant pas suffisant pour permettre au diffuseur d'assurer une qualité de prestation optimale.

### Objet et résumé de l'invention

La présente invention vise donc à améliorer le niveau de confort procuré par tel diffuseur de fragrance autonome en adaptant son fonctionnement suivant le contexte.

Elle propose à cet effet un procédé de diffusion de fragrance dans l'habitacle d'un véhicule automobile selon la revendication 1.

Le dispositif diffuseur selon l'invention permet ainsi d'atténuer voire de supprimer le bruit de fond généré par le pulseur d'air pendant les phases d'arrêt du moteur où ce bruit parasite est particulièrement dérangeant pour les passagers du véhicule car non couvert par celui habituel du moteur.

L'implantation d'un tel dispositif diffuseur est ainsi particulièrement avantageuse concernant les véhicules équipés de systèmes de type « Stop and Start » aptes à couper automatiquement le moteur suite à un arrêt prolongé du véhicule.

Selon des caractéristiques préférées du dispositif diffuseur selon l'invention, prises seules ou en combinaison :
- ledit pulseur est arrêté et en ce que lesdits moyens d'obturation ferment l'orifice de ladite cartouche pendant lesdites phases d'arrêt du moteur ;
- le ratio entre les durées d'ouverture et de fermeture de l'orifice de ladite cartouche sur un cycle de diffusion de la fragrance varie en fonction de la température extérieure audit véhicule ;
- ledit ratio est pondéré à la baisse lorsque la température extérieure est supérieure ou égale à une première température seuil ;
- ladite première température seuil est comprise entre 25 et 30°C
- ledit ratio est pondéré à la hausse lorsque la température extérieure est inférieure ou égale à une seconde température seuil ;
- ladite seconde température seuil est comprise entre 10 et 15°C ;
- ledit ratio est pondéré de manière systématique selon un coefficient déterminé selon une fonction décroissante de la température extérieure ; et/ou
- le ratio entre les durées d'ouverture et de fermeture dudit orifice sur un cycle de diffusion est pondéré à la hausse au fur et à mesure de l'augmentation de la durée d'utilisation de ladite cartouche.

L'invention vise également sous un second aspect un véhicule automobile comportant un tel dispositif diffuseur.

### Brève description des dessins

L'exposé de l'invention sera maintenant poursuivi par la description détaillée d'un exemple de réalisation, donnée ci-après à titre illustratif mais non limitatif, en référence aux dessins annexés, sur lesquels :
- la figure 1 est une représentation schématique du dispositif de diffusion de fragrance dans l'habitacle selon l'invention ; et
- la figure 2 est une vue en coupe du dispositif de diffusion de fragrance de la figure 1 prise selon le plan II-II.

### Description détaillée d'un mode de réalisation

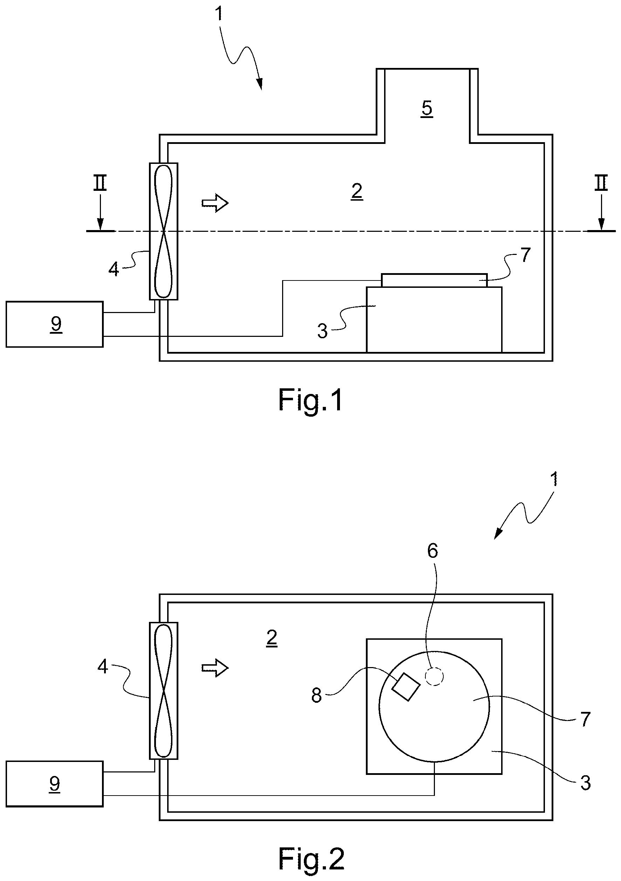

Le dispositif diffuseur de fragrance 1 représenté schématiquement sur la figure 1 est de type autonome, c'est-à-dire qu'il est apte à diffuser du parfum dans l'habitacle du véhicule indépendamment du système de ventilation et/ou de climatisation du véhicule, et au travers d'au moins un passage en forme de fente ménagé dans la paroi frontale de la planche de bord.

Il comporte un corps creux 2 encastré dans la planche de bord du véhicule et définissant un logement d'accueil pour au moins une cartouche 3 contenant un élément de stockage et de diffusion d'une fragrance constitué par exemple par une mèche ou des granulés poreux imprégnés d'un parfum liquide d'ambiance, d'une huile essentielle ou de tout autre produit olfactif approprié.

Le dispositif diffuseur de fragrance 1 comporte également un pulseur rotatif 4 apte à générer un flux d'air circulant dans le corps creux 2 avant d'être diffusé dans une conduite de sortie 5 en communication fluidique avec ce corps creux 2 et débouchant dans l'habitacle du véhicule au niveau du passage ménagé dans la planche de bord.

La cartouche 3 comporte un orifice 6 au travers duquel peut s'effectuer l'échange dans le corps creux 2 entre le flux d'air généré par le pulseur 4 et la fragrance contenue dans cette cartouche 3, de sorte que cette dernière soit diffusée de façon homogène dans l'habitacle.

Le dispositif diffuseur 1 comporte en outre des moyens d'obturation 7 aptes à gérer l'ouverture et la fermeture de l'orifice 6.

Ces moyens d'obturation se présentent avantageusement sous la forme d'un disque 7 muni d'une fenêtre 8 et entrainable à rotation par l'intermédiaire d'un actionneur électromagnétique entre :
- une position de fermeture dans laquelle il obstrue de manière étanche l'orifice 6 de la cartouche 3 de sorte à ce que la fragrance contenue dans cette cartouche 3 ne se mélange pas à l'air contenu dans le corps creux 2 et ne soit donc pas diffusée dans l'habitacle (configuration illustrée par la figure 2) ; et
- une position d'ouverture dans laquelle la fenêtre 8 coïncide avec l'orifice 6 de la cartouche 3 de sorte que ce dernier demeure ouvert afin de permettre le mélange de la fragrance correspondante avec l'air contenu dans le corps creux 2 et sa diffusion dans l'habitacle.

En variante, les moyens d'obturation sont différents, ces derniers étant par exemple constitués par un clapet monté pivotant entre une position de fermeture dans laquelle il obstrue de manière étanche l'orifice de la cartouche et une position d'ouverture où cet orifice demeure ouvert.

Le dispositif diffuseur 1 est relié à l'ordinateur de bord du véhicule auquel il transmet la référence de la cartouche 3 insérée dans le corps creux 2. En fonction de cette référence et d'un tableau de correspondance stocké en mémoire, l'ordinateur de bord peut ainsi déterminer la fragrance contenue dans cette cartouche.

Une interface de commande non représentée permet à l'utilisateur de mettre en route le dispositif diffuseur de fragrance 1 ou de l'arrêter.

Le dispositif diffuseur 1 comporte en outre un module de pilotage 9 commandant les moyens d'obturation 7 de sorte à gérer l'ouverture de l'orifice 6 de la cartouche 3 en fonction notamment de la température extérieure et de la durée d'utilisation de cette cartouche 3.

Les paramètres d'ouverture de cet orifice 6 sont déterminés via des formules de calcul et/ou des tableaux de correspondance stockés dans une mémoire du module de pilotage 9 et établis de sorte que le ressenti olfactif demeure sensiblement constant pour les passagers quelles que soient les conditions.

La température extérieure est obtenue à l'aide d'un capteur relié directement au module de pilotage 9 ou via le boîtier de servitude intelligent (BSI) du véhicule.

Lorsque cette température extérieure est supérieure ou égale à une première température seuil prédéterminée (comprise par exemple entre 25 et 30°C), le ratio entre les durées d'ouverture et de fermeture de l'orifice 6 de la cartouche 3 sur un cycle de diffusion (rapport cyclique de diffusion) est pondéré à la baisse (par exemple jusqu'à 50% de sa valeur nominale) afin de compenser la plus forte volatilité des molécules de fragrance dans de l'air chaud.

A l'inverse, lorsque la température extérieure est inférieure ou égale à une première température seuil prédéterminée (comprise par exemple entre 10 et 15°C), le ratio entre les durées d'ouverture et de fermeture de l'orifice 6 de la cartouche 3 sur un cycle de diffusion (rapport cyclique de diffusion) est pondéré à la hausse (par exemple jusqu'à 150 % de sa valeur nominale) de sorte à compenser la plus faible volatilité des molécules de fragrance dans de l'air froid.

La pondération du rapport cyclique de diffusion peut également être effectuée de manière systématique selon un coefficient déterminé selon une fonction décroissante de la température extérieure.

Ce rapport cyclique de diffusion est également pondéré à la hausse au fur et à mesure de l'augmentation de la durée d'utilisation de la cartouche 3 (cette durée étant déterminée par un compteur associé à la cartouche 3) pour palier au « vieillissement » de la fragrance contenu dans cette dernière.

Pendant les phases d'arrêt du moteur et alors que le véhicule demeure alimenté électriquement (cette coupure du moteur étant d'origine manuelle suite au positionnement par le conducteur de la clé sur la position « + Après contact » ou effectuée automatiquement par la fonction « Stop and Start » du véhicule suite à un arrêt prolongé de ce dernier), le module de pilotage 9 ordonne l'arrêt du pulseur 4 du dispositif diffuseur 1 de sorte à ne pas perturber les passagers du véhicule avec le bruit de fond généré par ce pulseur 4 et préalablement couvert par celui du moteur auquel ces passagers sont habitués.

Avantageusement, le module de pilotage 9 va également demander aux moyens d'obturation 7 de fermer l'orifice 6 de la cartouche 3 pour stopper la diffusion de la fragrance, de sorte à ne pas en consommer inutilement ainsi qu'à éviter qu'un air sursaturé en fragrance ne soit diffusé dans l'habitacle lors du redémarrage du pulseur 4.

Selon une variante de réalisation, le module de pilotage 9 ne coupe pas le pulseur 4 mais diminue simplement sa vitesse de rotation de sorte à limiter la nuisance sonore généré par ce dernier.

Dans un tel cas de figure, les moyens d'obturation 7 ne sont donc pas sollicités pour fermer l'orifice 6 de la cartouche 3 de sorte que la diffusion dans l'habitacle de la fragrance puisse se poursuivre.

Selon une variante, le dispositif peut comprendre plusieurs cartouches ou plusieurs compartiments contenant des fragrances différentes.

## Revendications

1. Procédé de diffusion d'au moins une fragrance dans l'habitacle d'un véhicule automobile à partir d'une cartouche (3) comportant un élément de stockage et de diffusion de ladite fragrance, la cartouche (3) étant logée dans
un dispositif diffuseur de fragrance (1) comportant un corps creux (2) définissant un logement d'accueil pour la cartouche (3), un pulseur rotatif (4) apte à générer un flux d'air circulant dans ledit corps creux (2) et délivré dans ledit habitacle par l'intermédiaire d'une conduite de sortie (5) en communication fluidique avec ledit corps creux (2), ladite cartouche (3) comprenant un orifice (6) à travers duquel peut s'effectuer l'échange dans ledit corps creux (2) entre le flux d'air généré par ledit pulseur (4) et ladite fragrance de sorte que ledit flux d'air délivré dans l'habitacle soit parfumé, ledit procédé consistant à arrêter ou réduire la vitesse du pulseur (4) pendant les phases durant lesquelles le moteur dudit véhicule est à l'arrêt alors que ledit véhicule demeure alimenté électriquement, **caractérisé en ce que** le dispositif (1) comprend en outre des moyens d'obturation (7) aptes à gérer l'ouverture et la fermeture de l'orifice (6) de ladite cartouche (3), consistant à pondérer à la hausse au fur et à mesure de l'augmentation de la durée d'utilisation de ladite cartouche (3) sur un cycle de diffusion, le ratio entre les durées d'ouverture et de fermeture de l'orifice (6) de ladite cartouche (3).

2. Procédé selon la revendication 1, **caractérisé en ce que** lorsque ledit pulseur (4) est arrêté, il consiste à commander lesdits moyens d'obturation (7) pour fermer l'orifice (6) de ladite cartouche (3) pendant lesdites phases d'arrêt du moteur.

3. Procédé selon l'une des revendications 1 ou 2, **caractérisé en ce que** le ratio entre les durées d'ouverture et de fermeture de l'orifice (6) de ladite cartouche (3) sur un cycle de diffusion de la fragrance varie en fonction de la température extérieure audit véhicule.

4. Procédé selon la revendication 3, **caractérisé en ce que** ledit ratio est pondéré à la baisse lorsque la température extérieure est supérieure ou égale à une première température seuil.

5. Procédé selon la revendication 4, **caractérisé en ce que** ladite première température seuil est comprise entre 25 et 30°C.

6. Procédé selon l'une des revendications 3 à 5, **caractérisé en ce que** ledit ratio est pondéré à la hausse lorsque la température extérieure est inférieure ou égale à une seconde température seuil.

7. Procédé selon la revendication 6, **caractérisé en ce que** ladite seconde température seuil est comprise entre 10 et 15°C.

8. Procédé selon la revendication 3, **caractérisé en ce que** ledit ratio est pondéré de manière systématique selon un coefficient déterminé selon une fonction décroissante de la température extérieure.

## Patentansprüche

1. Verfahren zum Diffundieren von mindestens einem Duftstoff in den Innenraum eines Kraftfahrzeugs von einer Kartusche (3), die ein Element zum Speichern und Diffundieren des Duftstoffs aufweist, wobei die Kartusche (3) in einer Duftdiffundiervorrichtung (1) untergebracht ist, die einen Hohlkörper (2), der ein Aufnahmegehäuse für die Kartusche (3) definiert, einen rotierenden Pulsator (4), der einen Luftstrom erzeugen kann, der in den Hohlkörper (2) strömt und über eine Auslassleitung (5) in Fluidverbindung mit dem Hohlkörper (2) in den Innenraum geliefert wird, aufweist, wobei die Kartusche (3) eine Öffnung (aufweist 6), durch die der Austausch in dem Hohlkörper (2) zwischen dem durch das Gebläse (4) erzeugten Luftstrom und dem Duftstoff erfolgen kann, sodass der in den Fahrgastraum gelieferte Luftstrom parfümiert ist, wobei das Verfahren darin besteht, die Geschwindigkeit des Gebläses (4) während der Phasen, in denen der Motor des Fahrzeugs steht, während das Fahrzeug elektrisch betrieben bleibt, zu stoppen oder zu verringern, **dadurch gekennzeichnet, dass** die Vorrichtung (1) ferner Verschlussmittel (7) umfasst, die geeignet sind, das Öffnen und Schließen der Öffnung (6) der Kartusche (3) zu steuern, wobei das Gewicht nach oben zu gewichten ist mit zunehmender Nutzungsdauer der Kartusche (3) über einen Diffusionszyklus das Verhältnis zwischen der Öffnungs- und Schließzeit der Öffnung (6) der Kartusche (3).

2. Verfahren nach Anspruch 1, **dadurch gekennzeichnet, dass**, wenn der Pulsator (4) angehalten wird, es darin besteht, die Verschlussmittel (7) zu steuern, um die Öffnung (6) der Patrone (3) während der Motorstillstandsphasen zu schließen.

3. Verfahren nach einem der Ansprüche 1 oder 2, **dadurch gekennzeichnet, dass** das Verhältnis zwischen den Öffnungs- und Schließzeiten der Öffnung (6) der Patrone (3) über einen Diffusionszyklus des Duftstoffs in Abhängigkeit von der Temperatur außerhalb des Fahrzeugs variiert.

4. Verfahren nach Anspruch 3, **dadurch gekennzeichnet, dass** das Verhältnis nach unten gewichtet wird, wenn die Außentemperatur größer oder gleich einer ersten Schwellentemperatur ist.

5. Verfahren nach Anspruch 4, **dadurch gekennzeichnet, dass** die erste Grenztemperatur zwischen 25 und 30°C liegt.

6. Verfahren nach einem der Ansprüche 3 bis 5, **dadurch gekennzeichnet, dass** das Verhältnis nach oben gewichtet wird, wenn die Außentemperatur kleiner oder gleich einer zweiten Grenztemperatur ist.

7. Verfahren nach Anspruch 6, **dadurch gekennzeichnet, dass** die zweite Grenztemperatur zwischen 10 und 15°C liegt.

8. Verfahren nach Anspruch 3, **dadurch gekennzeichnet, dass** das Verhältnis systematisch mit einem Koeffizienten gewichtet wird, der gemäß einer abnehmenden Funktion der Außentemperatur bestimmt wird.

## Claims

1. Process for the distribution of at least one fragrance in the passenger compartment of a motor vehicle from a cartridge (3) incorporating a storage and diffusion element of that fragrance, the cartridge (3) being housed in a fragrance diffuser (1) having a hollow body (2) defining a housing for the cartridge (3), a rotary puller (4) capable of generating a circulating air flow In that hollow body (2) and delivered in that passenger compartment via an outlet pipe (5) in fluidized communication with that hollow body (2), that cartridge (3) comprising an orifice (6) through which the exchange in that hollow body (2) can take place between the air flow generated by that pulsor (4) and that fragrance, so that that air flow delivered into the vehicle passenger compartment is perfumed, the process of stopping or reducing the speed of the pulsor (4) during the phases during which the engine of that vehicle is stationary while the vehicle remains electrically powered, **characterized by** the fact that the device (1) also includes means of sealing (7) capable of managing the opening and closing of the orifice (6) of that cartridge (3), consisting of increasing weight. and as the duration of use of that cartridge increases (3) over a diffusion cycle, the ratio of the opening and closing times of the orifice (6) of that cartridge (3).

2. Method according to claim 1, **characterized in that** when said pulsor (4) is stopped, it consists in controlling said closing means (7) to close the orifice (6) of said cartridge (3) during said engine stopping phases.

3. Method according to one of Claims 1 or 2, **characterized in that** the ratio between the opening and closing times of the orifice (6) of said cartridge (3) over a fragrance diffusion cycle varies as a function of the temperature outside said vehicle.

4. A method according to claim 3, **characterized in that** said ratio is weighted down when the outside temperature is greater than or equal to a first threshold temperature.

5. A method according to claim 4, **characterized in that** said first threshold temperature is between 25 and 30°C.

6. Method according to one of Claims 3 to 5, **characterized in that** the said ratio is weighted upwards when the outside temperature is less than or equal to a second threshold temperature.

7. A method according to claim 6, **characterized in that** said second threshold temperature is between 10 and 15°C.

8. Method according to claim 3, **characterized in that** said ratio is weighted systematically according to a coefficient determined according to a decreasing function of the outside temperature.
